(19) Europäisches Patentamt
European Patent Office
Office européen des brevets

(11) Veröffentlichungsnummer: **0 193 712**
B1

(12) **EUROPÄISCHE PATENTSCHRIFT**

(45) Veröffentlichungstag der Patentschrift:
25.04.90

(51) Int. Cl.⁴: **H04N 5/32**

(21) Anmeldenummer: **86100517.1**

(22) Anmeldetag: **16.01.86**

(54) Röntgendiagnostikeinrichtung für Subtraktionsangiographie.

(30) Priorität: **04.02.85 DE 3503722**

(43) Veröffentlichungstag der Anmeldung:
**10.09.86 Patentblatt 86/37**

(45) Bekanntmachung des Hinweises auf die Patenterteilung:
**25.04.90 Patentblatt 90/17**

(84) Benannte Vertragsstaaten:
**DE FR**

(56) Entgegenhaltungen:
**US-A- 4 204 225**

(73) Patentinhaber: **Siemens Aktiengesellschaft,
Wittelsbacherplatz 2, D-8000 München 2(DE)**

(72) Erfinder: **Pfeiler, Manfred, Dr. Dipl.-Ing.,
Ludwig-Thoma-Strasse 25, D-8520 Erlangen(DE)**
Erfinder: **Seissl, Johann, Grossgründlacher Strasse 20,
D-8520 Erlangen(DE)**
Erfinder: **Block, Peter C., Dr., 143 Meadowbrook Road,
Weston Massachusetts 02193(US)**

**Beschreibung**

Die Erfindung betrifft eine Röntgendiagnostikeinrichtung gemäß dem Oberbegriff des Patentanspruches 1. Derartige Röntgendiagnostikeinrichtungen dienen zum Erstellen von Aufnahmen für die Subtraktionsangiographie.

In der DE-A 3 124 583 ist eine derartige Röntgendiagnostikeinrichtung beschrieben, bei der in einem ersten Bildspeicher ein erstes, eventuell integriertes Bild einer ersten Herzphase eingelesen wird. Das Videosignal dieses ersten Bildes wird von den Videosignalen sämtlicher darauffolgenden Bilder in einer Differenzstufe subtrahiert. In einem zweiten Bildspeicher, der an die Differenzstufe angeschlossen ist, in der das aktuelle Videosignal und das gespeicherte, der ersten Herzphase entsprechende Videosignal voneinander subtrahiert werden, wird ein zu einer zweiten Herzphase gehörendes Differenzsignal eingespeichert. Dadurch lassen sich die maximale Ausdehnung und maximale Kontraktion eines Herzes und damit der Bewegungsraum des Organes erkennen. Eine solche Vorrichtung läßt sich aber bei der nachfolgend beschriebenen sogenannten Pfadfindertechnik nicht verwenden.

Aus der US-A 4 204 225 ist eine Echtzeit-Röntgen-Subtraktionsvorrichtung bekannt, bei der Videosignale über einen vollständigen Herzzyklus integriert und als Maskensignal von darauffolgenden Signalen, die über einen kleineren Zeitraum integriert wurden, subtrahiert werden. Die Integrationsdauer des Maskensignales wird dabei vom EKG abgeleitet. Auch diese Vorrichtung läßt sich nicht bei der nachfolgend beschriebenen Pfadfindertechnik verwenden, da in dem integrierten Maskensignal die Bewegungen verwischt sind.

Damit die untersuchende Person bei der Einführung eines Katheters in die Gefäßbahnen deren Verlauf auch ohne Kontrastmittel erkennen kann, wird die Pfadfindertechnik angewandt, bei der das laufende Durchleuchtungsbild, das den Katheter zeigt, mit der aus einer Subtraktionsangiographie erzeugten Gefäßdarstellung überlagert wird. Dadurch erkennt die untersuchende Person den Verlauf der Gefäße und kann den Katheter entsprechend steuern. Die Übernahme dieser Pfadfindertechnik für die Herz-Angiographie ist aber nicht möglich, weil die Subtraktionsangiographie ein statisches Gefäßbild erzeugt, das einem speziell in bezug auf das Herz dynamischen Durchleuchtungsbild überlagert wird. Dadurch stimmen die Konturen des bewegten Herzens nur zu einem einzigen Zeitpunkt, bzw. bei der Integration niemals mit den von dem Subtraktionsbild gelieferten Konturen überein.

Die Erfindung geht von der Aufgabe aus, eine Röntgendiagnostikeinrichtung gemäß dem Oberbegriff des Patentanspruches 1 zu schaffen, bei der die Pfadfindertechnik auch bei zyklischen dynamischen Vorgängen, beispielsweise beim Herzen, ermöglicht wird, ohne daß störende Bewegungsvorgänge die Sichtbarkeit erschweren.

Die Aufgabe ist bei einer Einrichtung der eingangs genannten Art erfindungsgemäß durch die im kennzeichnenden Teil des Patentanspruches 1 angegebenen Merkmale gelöst. Dadurch wird erreicht, daß im Durchleuchtungsbetrieb zum aktuellen Videosignal das zu gleicher Herzphase gehörende, gespeicherte Videosignal des Gefäßsystemes überlagert wird.

Ein vorteilhafter Aufbau der Röntgendiagnostikeinrichtung ergibt sich, wenn zwei Bildspeicher vorhanden sind, deren Ausgänge an einem Umschalter angeschlossen sind, der mit dem einen Eingang der Differenzstufe verbunden ist, deren zweitem Eingang das aktuelle Videosignal zugeführt ist, und wenn die Aufnahmesteuervorrichtung derart ausgebildet ist, daß sie zu Beginn der Untersuchung eine über wenigstens einen Herzzyklus integrierte Hilfsmaske in dem ersten Bildspeicher einspeichert, der gesteuert von der Aufnahmesteuervorrichtung über den Umschalter mit der Differenzstufe verbunden ist, daß nach der Injektion eines Kontrastmittels in Abhängigkeit vom Maximum des Kontrastmittelverlaufes im interessierenden Bereich mehrere aufeinanderfolgende, zu einem Herzzyklus gehörende Bilder in den zweiten Bildspeicher eingelesen werden, und daß nach erfolgter Einspeicherung der Schalter den zweiten Bildspeicher mit der Differenzstufe verbindet. Eine sichere Einspeicherung eines vollständigen Herzzyklus wird erreicht, wenn die Aufnahmesteuervorrichtung derart ausbildet ist, daß sie den für den Patienten typischen Herzzyklus ermittelt und eine Einspeicherung in den zweiten Bildspeicher nur dann bewirkt, wenn der Herzzyklus in seiner Periodendauer dem typischen Herzzyklus entspricht. Eine genauere Synchronisation der Herzphasen wird erreicht, wenn die Aufnahmesteuervorrichtung derart ausgebildet ist, daß zur Synchronisierung mehrere identifizierbare Merkmale enthaltende Abschnitte des EKGs ermittelt werden. Eine optimale Anpassung der Zeitdauer der einzelnen Herzphasen läßt sich erreichen, wenn die Aufnahmesteuervorrichtung derart ausgebildet ist, daß die Dauer der gespeicherten Herzphase durch Interpolation der Einzelbilder in der Zeitachse veränderbar ist.

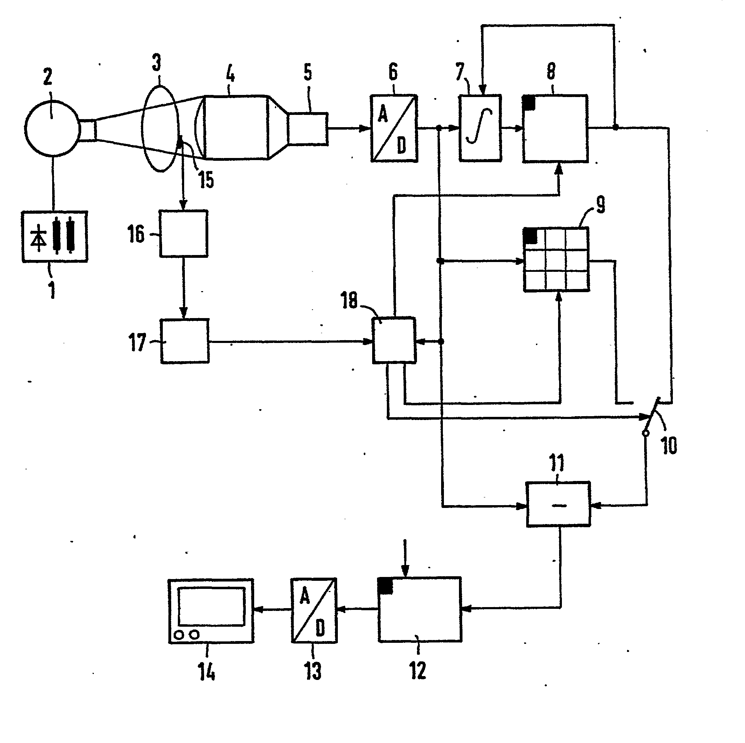

Die Erfindung wird nachfolgend anhand eines in der Figur dargestellten Ausführungsbeispieles näher erläutert.

In der Figur ist eine erfindungsgemäße Röntgendiagnostikeinrichtung mit einem Hochspannungsgenerator 1 dargestellt, der eine Röntgenröhre 2 speist, in derem Strahlengang sich ein Patient 3 befindet. Ein im Strahlengang nachfolgender Röntgenbildverstärker 4 ist mit einer Fernsehkamera 5 verbunden, deren Ausgangssignal einem Analog/-Digital-Wandler (A/D-Wandler 6) zugeführt ist. Das digitalisierte Videosignal wird über eine Integrationsstufe 7 in einen ersten Bildspeicher 8 eingelesen, dessen Ausgang auf den zweiten Eingang der Integrationsstufe 7 rückgekoppelt ist. Das Ausgangssignal des A/D-Wandlers 6 ist weiterhin mit einem zweiten Bildspeicher 9 verbunden, der eine Speicherkapazität von mehreren Einzelbildern aufweist. Die Ausgänge der Bildspeicher 8 und 9 sind über einen Umschalter 10 mit dem einen Eingang einer Differenzstufe 11 verbunden, an deren zweitem Eingang der A/D-Wandler 6 angeschlossen ist. Das Ausgangssignal der Differenzstufe 11 wird einem dritten Bildspeicher 12 zugeführt, dessen Ausgang mit einem Digital/Analog-Wandler (D/A-Wandler 13) verbunden ist, dessen analoges Ausgangssignal auf einem Monitor 14 dargestellt wird.

An dem Patienten 3 sind EKG-Elektroden 15 angebracht, die mit einer EKG-Schaltung 16, einem Verstärker mit Impedanzwandler, verbunden sind. Das EKG-Signal der EKG-Schaltung 16 wird einem Diskriminator 17 für die Amplitude und Phase des EKG-Signales zugeführt. Der Diskriminator 17 ist mit einer Steuereinheit 18 verbunden, die eine Einspeicherung in die beiden Bildspeicher 8 und 9 bewirkt. Die EKG-Schaltung 16, der Diskriminator 17 und die Steuereinheit 18 bilden die Aufnahmesteuervorrichtung 16 bis 18, die beispielsweise den nachfolgend beschriebenen Aufbau aufweisen können.

Der Diskriminator 17 kann beispielsweise aus einer Schwellenschaltung für die Amplitude und Phasenschiebern, beispielsweise monostabilen Kippstufen, bestehen. Hierdurch erhält man aus der R-Zacke des EKG-Signales zeitlich versetzte Steuerimpulse. Die Steuereinheit 18 kann beispielsweise aus UND-Schaltungen, Verzögerungsgliedern und Verriegelungsschaltungen aufgebaut sein. Hierbei werden den ersten Eingängen der UND-Glieder die Vertikalimpulse der Fernsehkamera zugeführt. Dem zweiten Eingang des ersten UND-Gliedes, das den Speicherplatz des zweiten Bildspeichers 9 steuert, wird das Ausgangssignal des Diskriminators 17 direkt zugeführt. Dem zweiten Eingang des zweiten UND-Gliedes, das den Speicherplatz des zweiten Bildspeichers 9 steuert, wird das Ausgangssignal des Diskriminators 17 über ein erstes Verzögerungsglied zugeleitet. Die weiteren Speicherplätze werden in gleicher Weise angesteuert. Den UND-Gliedern sind die Verriegelungsglieder nachgeschaltet, die beispielsweise eine mehrfache Auslösung der Speicherung an einem Speicherplatz verhindern. Die Verriegelungsglieder können aus bistabilen Kippstufen aufgebaut sein.

Eine weitere Möglichkeit der Aufnahmesteuerung kann auch dadurch erreicht werden, daß der Diskriminator 17 mehrere Diskriminatorschaltungen aufweist und eine erste Diskriminatorschaltung auf die R-Zacke, eine zweite Diskriminatorschaltung auf die T-Welle und weitere Diskriminatorschaltungen auf weitere signifikante Merkmale des EKG-Signals ansprechen. Diese Ausgangssignale könnten den Bildspeicher 9 über Verriegelungsglieder direkt zugeführt werden. Es kann aber auch eine Kombination von mehreren Diskriminatorschaltungen und Verzögerungsgliedern Verwendung finden.

Nach Einschaltung der Durchleuchtung liegen am Ausgang des A/D-Wandlers 6 die digitalen Videosignale an. Diese werden über wenigstens einen Herzzyklus integriert als Hilfsmaske in den ersten Bildspeicher 8 eingelesen. Der Umschalter 10 verbindet den Ausgang des ersten Bildspeichers 8 mit der Differenzstufe 11. Anschließend wird dem Patienten 3 ein Kontrastmittel injiziert, das mit einer kleinen Zeitverschiebung im interessierenden Bereich in dem Subtraktionsbild auf dem Monitor 14 zu sehen ist. Die Einspeicherung in den zweiten Bildspeicher 9 wird so automatisch oder von Hand ausgelöst, daß die Kontrastmittelkonzentration der eingespeicherten Bilder ein Maximum erreicht. Hier werden nun nach Auftreten eines Erkennungsmerkmales des EKG's, beispielsweise die R-Zacke, nacheinander, zu unterschiedlichen Herzphasen eines Herzzyklus gehörende Aufnahmen als Maskenzyklus eingespeichert. Nach Beendigung der Einspeicherung wird der Umschalter 10 durch die Steuervorrichtung 18 betätigt, so daß der Ausgang des zweiten Bildspeichers 9 mit der Differenzstufe 11 verbunden ist. Die in diesem zweiten Bildspeicher 9 enthaltenen Einzelbilder werden herzphasensynchron mit dem aktuellen Videosignal ausgelesen. Dadurch erscheint am Monitor das Gefäßsystem als helle, dem übrigen Bildinhalt überlagerte Abbildung. In den derart sichtbar gemachten Gefäßen kann nun der Katheter eingeführt und richtig geleitet werden. Durch das Auslesen des Bildspeichers 9 herzphasensynchron mit dem aktuellen Videosignal werden einander entsprechende Einzelbilder subtrahiert, so daß in jedem Fall gewährleistet ist, daß die aus dem gespeicherten Bild abgeleiteten Gefäße den wirklichen Konturen entsprechen.

Soll der vollständige Herzzyklus in den zweiten Bildspeicher 9 eingelesen werden, so daß eine sehr genaue Überlagerung erfolgen kann, so muß er eine Speicherkapazität von etwa 50 bis 60 Halbbildern aufweisen. Es kann aber auch die Speicherkapazität reduziert werden, wenn beispielsweise nur jedes zweite oder dritte Halbbild abgespeichert ist, so daß sich noch hinreichend genaue Abbildungen des Herzzyklus ergeben.

Da aber die Herzzyklen nicht immer gleich sind, beispielsweise bei Auftreten einer Extrasystole, ermittelt die Steuervorrichtung 18 vor Einspeicherung des Maskenzyklus in den zweiten Bildspeicher 9 die Länge eines durchschnittlichen typischen Herzzyklus. Beginnt nun die Einspeicherung in dem zweiten Bildspeicher 9, so wird die Dauer des aktuellen Herzzyklus mit der typischen Dauer verglichen. Bei Gleichheit der Dauer wird der Einspeichervorgang abgeschlossen. Ist dagegen eine Abweichung der Dauer des aktuellen vom typischen Herzzyklus festzustellen, so werden die alten Bilder gelöscht und der nächste Herzzyklus eingespeichert.

Bei der Wiedergabe wird der Beginn des Auslesens des zweiten Bildspeichers 9 beispielsweise immer mit der R-Zacke des EKG's synchronisiert. Unterschiede in der Periodendauer werden durch Wegfall der letzten Bilder bzw. mehrmaliges Auslesen derselben ausgeglichen. Wird aber beispielsweise bei der Einspeicherung bereits der Herzzyklus in mehrere Segmente unterteilt, d.h. er wird beispielsweise auf mehrere identifizierbare Merkmale des EKG's synchronisiert, so kann dies auch bei der Wiedergabe erfolgen. Dadurch wird erreicht, daß ein Ausgleich der Dauer bereits in den Segmenten erfolgt, so daß die dynamische Darstellung der Gefäße dem Original besser entspricht. Die Dauer des Herzzyklus kann aber auch durch Interpolation der gespeicherten Einzelbilder in der Zeitachse an den aktuellen Herzzyklus angeglichen werden.

Der erste Bildspeicher 8 kann entfallen, wenn die Hilfsmaske entsprechend integriert in einen Speicherplatz des zweiten Bildspeichers 9 eingelesen wird. Wird nun die Einspeicherung des Herzzyklus ausgelöst, so wird diese Hilfsmaske nicht mehr benötigt, so daß dieser Speicherplatz überschrieben werden kann. Dadurch kann auch der Umschalter 10 entfallen, so daß der Bildspeicher 9 immer mit der Differenzstufe 11 verbunden ist. Der dritte Bildspeicher 12, der vorzugsweise ein Massenspeicher ist, dient zur Aufnahme einzelner Szenen. Er wird ebenfalls für die erfindungsgemäße Röntgendiagnostikeinrichtung nicht unbedingt benötigt.

**Patentansprüche**

1. Röntgendiagnostikeinrichtung mit einer Röntgenbildverstärker-Fernsehkette (4, 5), einem Bildspeicher (9) und einer Differenzstufe (11), die gespeicherte und den gespeicherten nachfolgende Videosignale voneinander subtrahiert, mit einer Steuervorrichtung (15 bis 18) für den Bildspeicher (9) zur Erzeugung von Steuerimpulsen aus dem EKG eines Patienten (3) für die Einspeicherung von Bildern aufweist, dadurch gekennzeichnet, daß der Bildspeicher (9) Speicherplätze für mehrere Einzelbilder aufweist, die während eines Herzzyklus des Patienten (3) entstehen, und daß die Steuervorrichtung (15 bis 18) aus einem von dem EKG abgeleiteten Signal mehrere zeitlich versetzte Steuersignale bei vorbestimmten, unterschiedlichen Herzphasen eines Herzzyklus erzeugt, die den Bildspeicher (9) derart steuern, daß in der Differenzstufe (11) zueinander gehörende Einzelbilder einer Herzphase innerhalb des Herzzyklus entsprechend überlagert werden.

2. Röntgendiagnostikeinrichtung nach Anspruch 1, dadurch gekennzeichnet, daß zwei Bildspeicher (8, 9) vorhanden sind, deren Ausgänge an einem Umschalter (10) angeschlossen sind, der mit dem einen Eingang der Differenzstufe (11) verbunden ist, deren zweitem Eingang das aktuelle Videosignal zugeführt ist, und daß die Aufnahmesteuervorrichtung (16 bis 18) derart ausgebildet ist, daß sie zu Beginn der Untersuchung eine über wenigstens einen Herzzyklus integrierte Hilfsmaske in dem ersten Bildspeicher (8) einspeichert, der gesteuert von der Aufnahmesteuervorrichtung (16 bis 18) über den Umschalter (10) mit der Differenzstufe (11) verbunden ist, daß nach der Injektion eines Kontrastmittels in Abhängigkeit vom Maximum des Kontrastmittelverlaufes im interessierenden Bereich mehrere aufeinanderfolgende, zu einem Herzzyklus gehörende Bilder in den zweiten Bildspeicher (9) eingelesen werden, und daß nach erfolgter Einspeicherung der Schalter (10) den zweiten Bildspeicher (9) mit der Differenzstufe (11) verbindet.

3. Röntgendiagnostikeinrichtung nach Anspruch 1 oder 2, dadurch gekennzeichnet, daß die Aufnahmesteuervorrichtung (16 bis 18) derart ausgebildet ist, daß sie den für den Patienten (3) typischen Herzzyklus ermittelt und eine Einspeicherung in dem zweiten Bildspeicher (9) nur dann bewirkt, wenn der Herzzyklus in seiner Periodendauer dem typischen Herzzyklus entspricht.

4. Röntgendiagnostikeinrichtung nach einem der Ansprüche 1 bis 3, dadurch gekennzeichnet, daß die Aufnahmesteuervorrichtung (16 bis 18) derart ausgebildet ist, daß zur Synchronisierung mehrere identifizierbare Merkmale enthaltende Abschnitte des EKG's ermittelt werden.

5. Röntgendiagnostikeinrichtung nach einem der Ansprüche 1 bis 4, dadurch gekennzeichnet, daß die Aufnahmesteuervorrichtung (16 bis 18) derart ausgebildet ist, daß die Dauer des gespeicherten Herzzyklus durch Interpolation der Einzelbilder in der Zeitachse veränderbar ist.

**Claims**

1. An x-ray diagnostics device with an x-ray image intensifier video chain (4, 5), an image memory (9) and a differencing stage (11), which subtracts stored video signals and subsequent video signals from each other, the device having, for the storage of images, a control device (15 to 18) for the image memory (9) for generating control pulses from the EKG of a patient (3), characterised in that the image memory (9) has memory locations for several individual images which form during a heart cycle of the patient (3), and in that the control device (15 to 18) generates from one of the signals discharged from the EKG several temporally staggered controls signals with predetermined, varying heart phases of a heart cycle, which control the image memory (9) such that in the differencing stage (11) individual images, belonging to each other, of one heart phase are correspondingly superimposed within the heart cycle.

2. An x-ray diagnostics device according to claim 1, characterised in that two image memories (8, 9) are present, the outputs of which are attached to a changeover switch (10), which is connected to the input of the differencing stage (11), the second input of which is supplied with the current video signal, and in that the pick-up control device (16 to 18) is constructed such that at the beginning of the examination it stores in the first image memory (8) an auxiliary mask integrated over at least one heart cycle, which, controlled by the pick-up control device (16 to 18), is connected by way of the changeover switch (10) to the differencing stage (11), in that after the injection of a contrast means, in dependence on the maximum of the contrast means characteristic in the region of interest, several images, following each other and belonging to one heart cycle, are read into the second image memory (9), and in that after resulting storage, the switch (10) connects the second image memory (9) to the differencing stage (11).

3. An x-ray diagnostics device according to claim 1 or 2, characterised in that the pick-up control device (16 to 18) is constructed such that it determines the typical heart cycle for the patient (3) and only effects storage in the second image memory (9), if the heart cycle corresponds in its cycle with the typical heart cycle.

4. An x-ray diagnostics device according to one of claims 1 to 3, characterised in that the pick-up control device (16 to 18) is constructed such that, for synchronising, sections of the EKG containing several identifiable features are detected.

5. A x-ray diagnostics device according to one of claims 1 to 4, characterised in that the pick-up control device (16 to 18) is constructed such that the duration of the stored heart cycle is able to be altered by interpolation of the individual images in the time axis.

## Revendications

1. Appareil de radiodiagnostic comportant une chaîne (4, 5) à amplificateur de brillance et caméra de télévision, une mémoire d'images (9) et un étage soustracteur (11), qui soustrait l'un de l'autre le signal vidéo mémorisé et le signal vidéo suivant mémorisé, et un dispositif de commande (15 à 18) pour la mémoire d'images (9) pour la production d'impulsions de commande à partir de l'électrocardiogramme d'un patient (3) pour la mémorisation d'images, caractérisé par le fait que la mémoire d'images (9) comporte des emplacements de mémoire pour plusieurs images individuelles qui apparaissent pendant un cycle cardiaque du patient (3), et que le dispositif de commande produit, à partir d'un signal dérivé de l'électrocardiogramme, plusieurs signaux de commande décalés dans le temps, lors de différentes phases prédéterminées d'un cycle cardiaque, ces signaux commandant les mémoires d'images (9) de telle sorte que des images individuelles, associées entre elles, d'une phase cardiaque au cours du cycle cardiaque, sont superposées de façon correspondante dans l'étage soustracteur (11).

2. Appareil de radiodiagnostic suivant la revendication 1, caractérisé par le fait qu'il est prévu deux mémoires d'images (8, 9), dont les sorties sont raccordées à un commutateur (10), qui est relié à une entrée de l'étage soustracteur (1), à l'autre entrée duquel est envoyé le signal vidéo actuel, et que le dispositif (16 à 18) de commande de prise de vues est agencé de telle sorte qu'il mémorise, au début de la recherche, un masque auxiliaire intégré pendant au moins un cycle cardiaque, dans la première mémoire d'images (8), qui est reliée, d'une manière commandée par le dispositif (16 à 18) de commande de prise de vues, par l'intermédiaire du commutateur (10), à l'étage soustracteur (11), qu'après l'injection d'un agent de contraste, plusieurs images successives, associées à un cycle cardiaque, sont mémorisées dans la seconde mémoire d'images (9), en fonction du maximum de la répartition de l'agent de contraste dans la région à laquelle on s'intéresse, et qu'une fois cette mémorisation réalisée, le commutateur (10) relie la seconde mémoire d'images (9) à l'étage soustracteur (11).

3. Appareil de radiodiagnostic suivant la revendication 1 ou 2, caractérisé par le fait que le dispositif (16 à 18) de commande de prise de vues est agencé de telle sorte qu'il détermine le cycle cardiaque typique pour le patient (3) et déclenche une mémorisation dans la seconde mémoire d'images (9) uniquement lorsque le cycle cardiaque correspond, du point de vue de la durée de sa période, au cycle cardiaque typique.

4. Appareil de radiodiagnostic suivant l'une des revendications 1 à 3, caractérisé par le fait que le dispositif (16 à 18) de commande de prise de vues est agencé de telle sorte que, pour la synchronisation, on détermine des sections de l'électrocardiogramme qui contiennent plusieurs caractéristiques identifiables.

5. Appareil de radiodiagnostic suivant l'une des revendications 1 à 4, caractérisé par le fait que le dispositif (16 à 18) de commande de prise de vues est agencé de telle sorte que la durée du cycle cardiaque mémorisé peut être modifiée par interpolation des images individuelles sur l'axe des temps.